# EUROPÄISCHE PATENTANMELDUNG

(11) **EP 3 636 645 A1**
(43) Veröffentlichungstag der Anmeldung: **15.04.2020**
(21) Anmeldenummer: 18199887.3
(22) Anmeldetag: 11.10.2018
(51) Int. Cl.: C07D 471/04

(54) **VERFAHREN ZUR HERSTELLUNG SCHWEFEL-SUBSTITUIERTER PYRIDINDERIVATE**

(71) Anmelder: Bayer Aktiengesellschaft, 51373 Leverkusen (DE)
(72) Erfinder: WILLOT, Matthieu, 40215 Düsseldorf (DE); MOSRIN, Marc, 50935 Köln (DE); FISCHER, Rüdiger, 50259 Pulheim (DE); HAGER, Dominik, 40789 Monheim (DE); HOFFMEISTER, Laura, 40593 Düsseldorf (DE)
(74) Vertreter: BIP Patents

(57) **Zusammenfassung**

Die vorliegende Erfindung betrifft ein Verfahren zur Herstellung von Schwefel-substituierten Pyridinderivaten der Formel (II) ausgehend von Verbindungen der Struktur Q-H über Zwischenstufen der Formel (IIIa) oder (IIIb) 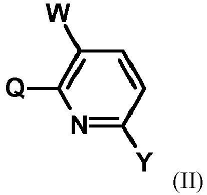 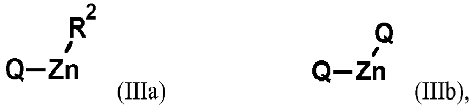 in welchen
Q für ein Strukturelement 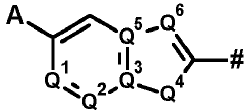 steht, wobei das Zeichen # die Bindung zum Rest des Moleküls anzeigt und A, Q¹, Q², Q³, Q⁴, Q⁵ und Q⁶ die in der Beschreibung angegebenen Bedeutungen haben,
W für S(O)ₙR⁸ steht, wobei
R⁸ für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-Cs)Cycloalkyl steht und
n für 0, 1 oder 2 steht,
Y für Wasserstoff, Halogen, CO₂R¹ oder NO₂ steht, wobei R¹ für (C₁-C₆)-Alkyl oder (C₁-C₆)-Haloalkyl steht und
R² für Halogen oder -O-Pivaloyl steht.

## Beschreibung

Die vorliegende Erfindung betrifft ein Verfahren zur Herstellung von Schwefel-substituierten Pyridinderivaten der Formel (II) 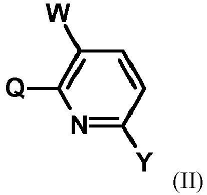 ausgehend von Verbindungen Q-H über Zwischenstufen der Formel (IIIa) oder (IIIb) 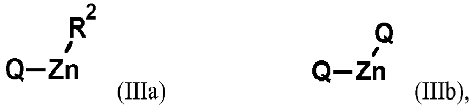 in denen die in den Formeln (II), (IIIa) und (IIIb) angegebenen Strukturelemente die nachstehend angegebenen Bedeutungen haben. Ferner betrifft die Erfindung derartige Schwefel-substituierte Pyridinderivate und Zwischenstufen.

Schwefel-substituierte Pyridinderivate der Formel (II) haben große technische Bedeutung für die pharmazeutische und die agrochemische Industrie und sind eine wichtige Zwischenstufe unter anderem bei der Herstellung von Verbindungen, die beispielsweise als Pestizide wirksam sind.

In der Literatur ist bekannt, dass Verbindungen der Formel (II) beispielsweise in einem ersten Schritt durch Kondensation von Pyridin-2-carbonsäurederivaten mit ortho-substituierten Bis(amin)-, Aminalkohol- oder Aminthiol-(hetero)arylderivaten in Gegenwart eines Kondensationsmittels (vgl. US 2003/069257 oder WO 2006/065703) und anschließend in einem zweiten Schritt durch weitere Kondensation wie in WO 2012/086848 beschrieben hergestellt werden können. Die bisher im Stand der Technik beschriebenen chemischen Syntheseverfahren von derartigen Schwefel-substituierten Pyridinderivaten bedienen sich aber sehr häufig Methoden, die aus industrieller Sicht wirtschaftlich nicht zu realisieren sind und/oder andere Nachteile aufweisen.

Nachteilig sind die geringen chemischen Ausbeuten, die Durchführung bei sehr hohen Temperaturen (ca. 150 °C bis 250 °C) sowie die mögliche schwierige Regio- und Chemo-Selektivität der Kondensation, insbesondere bei Imidazopyridin- und Imidazopyridazinderivaten. Die Herstellung ist daher sehr teuer und nicht für großtechnische kommerzielle Verfahren geeignet. Außerdem sind entsprechende Verbindungen kaum kommerziell erhältlich. Dies gilt insbesondere für 6-substituierte 3-alkylsulfanylpyridin-2-carbonsäurederivate.

Im Hinblick auf die vorstehend geschilderten Nachteile besteht dringend Bedarf für ein vereinfachtes, technisch und ökonomisch durchführbares Verfahren zur Herstellung von Schwefel-substituierten Pyridinderivaten, insbesondere von Schwefel-substituierten Pyridinderivaten der Formel (II). Die mit diesem angestrebten Verfahren erhältlichen Schwefel-substituierten Pyridinderivate sollen dabei vorzugsweise mit guter Ausbeute, hoher Reinheit und in ökonomischer Weise erhalten werden.

Überraschenderweise wurde gefunden, dass in einem Verfahren unter Verwendung einer Zink-metallorganischen Base Schwefel-substituierte Pyridinderivate der Formel (II) vorteilhaft hergestellt werden können.

Gegenstand der vorliegenden Erfindung ist demnach ein Verfahren zur Herstellung von Verbindungen der Formel (II) 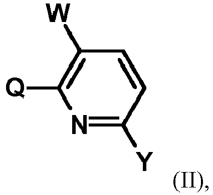 in welcher (Ausgestaltung 1)
- Q: für ein Strukturelement 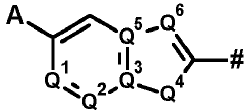
steht, wobei das Zeichen # die Bindung zum Rest des Moleküls anzeigt und
- Q¹: für N oder CR⁶ steht,
- Q²: für N oder CR⁶ steht,
- Q³: für N oder C steht,
- Q⁴: für O, S, N, CR⁶ oder NR⁷ steht,
- Q⁵: für N oder C steht und
- Q⁶: für N oder CH steht,
wobei maximal fünf der Variablen Q¹, Q², Q³, Q⁴, Q⁵ und Q⁶ gleichzeitig für Stickstoff stehen und Q³ und Q⁵ nicht gleichzeitig für N stehen und
- R⁶: für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁**-**C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
- R⁷: für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
- A: für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht, oder
- A: für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
- W: für S(O)ₙR⁸ steht, wobei
- R⁸: für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
- n: für 0, 1 oder 2 steht und
- Y: für Wasserstoff, Halogen, CO₂R¹ oder NO₂ steht, wobei R¹ für (C₁-C₆)-Alkyl oder (C₁-C₆)-Haloalkyl steht,
dadurch gekennzeichnet, dass in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 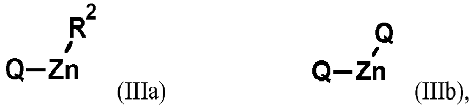 in welcher Q und R² jeweils die oben genannten Bedeutungen haben,
und diese Verbindung der Formel (IIIa) oder (IIIb) in einem zweiten Verfahrensschritt b) mit einer Verbindung der Formel (I) 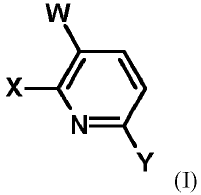 in welcher X für Halogen steht und W und Y jeweils die oben genannten Bedeutungen haben, in Gegenwart eines Katalysators zur Verbindung der Formel (II) umgesetzt wird.

Vorzugsweise stehen maximal vier der Variablen Q¹, Q², Q³, Q⁴, Q⁵ und Q⁶ gleichzeitig für Stickstoff. Unter Stickstoff werden hierbei N und/oder NR⁷ verstanden.

Bevorzugte und besonders bevorzugte Bedeutungen der in den vorstehend erwähnten Formeln (I), (II) (IIIa) und (IIIb) des erfindungsgemäßen Verfahrens aufgeführten Reste Q, W, R¹, R², X, und Y werden im Folgenden erläutert, wobei die Zink-metallorganische Base weiter unten noch genau beschrieben wird, so dass die bevorzugten Ausgestaltungen der Base dort angegeben sind.

### (Ausgestaltung 2)

- Q: steht bevorzugt für ein Strukturelement aus der Reihe Q1 bis Q15, 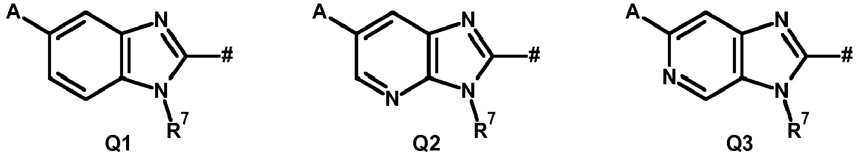 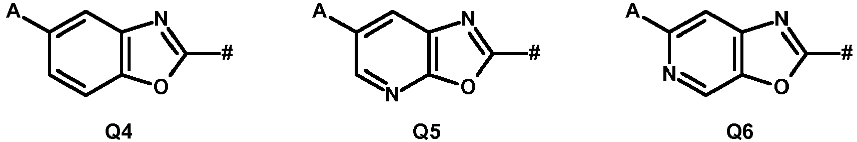 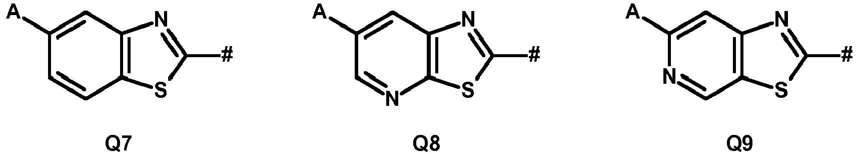 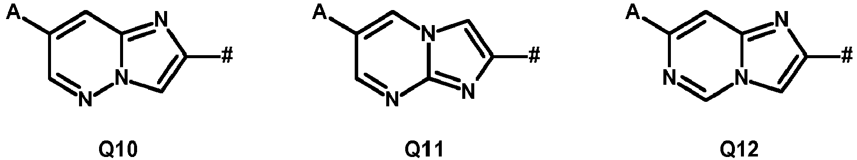 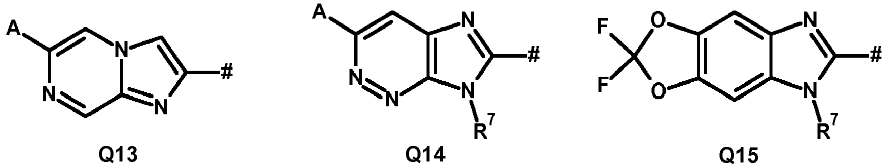
, wobei
- R⁷: bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
- A: bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
- W: steht bevorzugt für S(O)ₙR⁸, wobei
- R⁸: bevorzugt für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
- n: bevorzugt für 0, 1 oder 2 steht,
- R²: steht bevorzugt für Halogen, insbesondere Chlor, Brom oder Iod,
- X: steht bevorzugt für Halogen, insbesondere Brom oder Iod und
- Y: steht bevorzugt für Wasserstoff, Fluor, Chlor, Brom, CO₂R¹ oder NO₂, wobei R¹ für (C₁-C₄)-Alkyl steht.

### (Ausgestaltung 3)

- Q: steht besonders bevorzugt für ein Strukturelement aus der Reihe Q2, Q3, Q10, Q12, Q14 oder Q15, wobei
- R⁷: besonders bevorzugt für (C₁-C₄)Alkyl oder (C₁-C₄)Alkoxy-(C₁-C₄)alkyl steht,
- A: besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
- W: steht besonders bevorzugt für S(O)ₙR⁸, wobei
- R⁸: besonders bevorzugt für (C₁-C₃)Alkyl oder (C₁-C₃)Halogenalkyl steht und
- n: besonders bevorzugt für 0, 1 oder 2 steht,
- R²: steht besonders bevorzugt für Chlor,
- X: steht besonders bevorzugt für Brom oder Iod und
- Y: steht besonders bevorzugt für Wasserstoff, Chlor, Brom, CO₂R¹ oder NO₂, wobei R¹ für (C₁-C₄)-Alkyl steht.

### (Ausgestaltung 4)

- Q: steht ganz besonders bevorzugt für das Strukturelement Q3 oder Q14, wobei
- R⁷: ganz besonders bevorzugt für Methyl oder Ethyl, insbesondere Methyl steht und
- A: ganz besonders bevorzugt für Trifluormethyl steht,
- W: steht ganz besonders bevorzugt für S(O)ₙR⁸, wobei
- R⁸: ganz besonders bevorzugt für Ethyl oder Trifluormethyl steht und
- n: ganz besonders bevorzugt für 0 steht,
- R²: steht ganz besonders bevorzugt für Chlor,
- X: steht ganz besonders bevorzugt für Brom oder Iod und
- Y: steht ganz besonders bevorzugt für Wasserstoff, Chlor, Brom, CO₂R¹ oder NO₂, wobei R¹ für Methyl steht.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

In einer weiteren bevorzugten Ausführungsform der Erfindung steht Q für Q¹ und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 5).

In einer weiteren bevorzugten Ausführungsform der Erfindung steht Q für Q2 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 6).

In einer weiteren bevorzugten Ausführungsform der Erfindung steht Q für Q3 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 7).

In einer weiteren bevorzugten Ausführungsform der Erfindung steht Q für Q4 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 8).

In einer weiteren bevorzugten Ausführungsform der Erfindung steht Q für Q5 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 9).

In einer weiteren bevorzugten Ausführungsform der Erfindung steht Q für Q6 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 10).

In einer weiteren bevorzugten Ausführungsform der Erfindung steht Q für Q7 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 11).

In einer weiteren bevorzugten Ausführungsform der Erfindung steht Q für Q8 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 12).

In einer weiteren bevorzugten Ausführungsform der Erfindung steht Q für Q9 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 13).

In einer weiteren bevorzugten Ausführungsform der Erfindung steht Q für Q10 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 14).

In einer weiteren bevorzugten Ausführungsform der Erfindung steht Q für Q11 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 15).

In einer weiteren bevorzugten Ausführungsform der Erfindung steht Q für Q12 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 16).

In einer weiteren bevorzugten Ausführungsform der Erfindung steht Q für Q13 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 17).

In einer weiteren bevorzugten Ausführungsform der Erfindung steht Q für Q14 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 18).

In einer weiteren bevorzugten Ausführungsform der Erfindung steht Q für Q15 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 19).

In einer besonders bevorzugten Ausführungsform der Erfindung steht Q für Q2, Q3, Q10, Q12, Q14 oder Q15 und R⁷, A, W, R², X, V und Y haben die in Ausgestaltung 1 oder die in Ausgestaltung 2 oder die in Ausgestaltung 4 angegebenen Bedeutungen (Ausgestaltung 20).

In einer ganz besonders bevorzugten Ausführungsform der Erfindung steht Q für Q3 oder Q14 und R⁷, A, W, R², X, V und Y haben die in Ausgestaltung 1 oder die in Ausgestaltung 2 oder die in Ausgestaltung 3 angegebenen Bedeutungen (Ausgestaltung 21).

In einer weiteren bevorzugten Ausführungsform der Erfindung steht W für S(O)ₙR⁸, wobei n für 0 oder 2 und R⁸ für (C₁-C₃)Alkyl oder (C₁-C₃)Halogenalkyl steht und Q, R⁷, A, R², X, V und Y haben die in Ausgestaltung 1 oder die in Ausgestaltung 2 oder die in Ausgestaltung 4 angegebenen Bedeutungen (Ausgestaltung 22).

Vorteilhafterweise lassen sich die halogenierten Pyridinderivate der Formel (II) mit dem erfindungsgemäßen Verfahren mit guten Ausbeuten und in hoher Reinheit herstellen. Ein großer Vorteil des erfindungsgemäßen Verfahrens ist dessen Regioselektivität. Aufgrund der sehr guten funktionellen Gruppen-Toleranz von Zink-Reagenzien sind Zinkbasen sehr attraktiv. Insbesondere vorteilhaft ist ferner die Möglichkeit, auch bei deutlich niedrigeren Temperaturen Negishi-Kupplungen durchführen zu können, wobei in erfindungsgemäßen Verfahren auch bei höheren Temperaturen empfindliche funktionelle Gruppen wie Ester oder Fluoratome toleriert werden, ohne die die vorhandene Regioselektivität zu beeinträchtigen. Ferner können Negishi Kreuzkupplungen im Rahmen eines erfindungsgemäßen Verfahrens auch in Gegenwart von ortho-Substituenten am Pyridingerüst gute Ausbeuten an Zielprodukt ergeben, obwohl solche Kupplungen mit 2-substituierten Pyridinderivaten bislang dafür bekannt sind, niedrige Ausbeuten zu liefern. Somit werden weitere und/oder flexiblere Derivatisierungen von Edukt und Produkt möglich, ohne Synthesenrouten ständig ändern bzw. anzupassen zu müssen.

Das erfindungsgemäße Verfahren kann anhand des folgenden Schemas (I) erläutert werden:

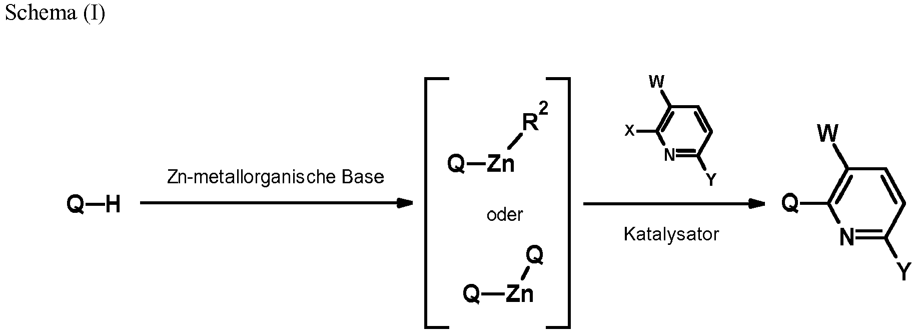

Hierin haben Q, W, R², X, und Y sowie die innerhalb der jeweiligen Definitionen ggfs. vorhandenen weiteren Strukturelemente jeweils die vorstehend angegebenen Bedeutungen. Die in Klammern angegebenen Verbindungen stellen die Zwischenstufe (Formel IIIa beziehungsweise IIIb) dar, welche mit einer Verbindung der Formel (I) weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung Q-H zur jeweiligen Zwischenstufe und Schritt b) die weitere Umsetzung der Zwischenstufe zur Verbindung der Formel (II) ist.

### Allgemeine Definitionen

Im Zusammenhang mit der vorliegenden Erfindung umfasst der Begriff Halogen (Hal), soweit nicht anders definiert, solche Elemente, die ausgewählt sind aus der Gruppe bestehend aus Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Der Begriff "Pivaloyl" beschreibt im Zusammenhang mit der vorliegenden Erfindung den deprotonierten Rest der Pivalinsäure (X) mit der Summenformel (CH₃)₃CCO₂H.

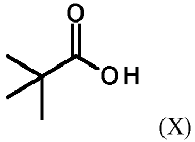

"O-Pivaloyl" bedeutet entsprechend, dass die Bindung des Pivaloylrestes über das deprotonierte Sauerstoffatom der Säuregruppe erfolgt.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Soweit nicht anders definiert steht Halogen dabei für Fluor, Chlor, Brom oder Iod, insbesondere für Fluor, Chlor oder Brom. Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂ oder CF₃CCl₂.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die Synthese von Verbindungen Q-H als Edukte eines erfindungsgemäßen Verfahrens ist dem Fachmann grundsätzlich bekannt. Beispielsweise können Verbindungen Q-H mit Q = Q1, Q2, Q3, Q14 oder Q15 aus entsprechenden (Het)Aryl-Diaminderivaten durch Ringschluss zur jeweiligen Imidazolverbindung erhalten werden, wie zum Beispiel in WO 2014/100065 oder WO 2015/017610 beschrieben, vorzugsweise unter sauren Bedingungen. Alternativsynthesen sind ebenfalls möglich, sind jedoch komplexer und dadurch im Regelfall wirtschaftlich nachteiliger. Die Verbindungen Q-H mit Q = Q4, Q5 oder Q6 können beispielweise aus entsprechenden (Het)Aryl-Aminoalkoholderivaten durch Ringschluss mit einem Orthoester der Ameisensäure zur jeweiligen Oxazolverbindung erhalten werden, wie zum Beispiel in WO 2018/037223. Die Verbindungen Q-H mit Q = Q7, Q8 oder Q9 können beispielweise aus entsprechenden (Het)Aryl-Aminohalogenderivaten durch Ringschluss mit einem O-Alkylcarbonothioat und anschließende Decarboxylierung zur jeweiligen Thiazolverbindung erhalten werden, wie zum Beispiel in WO 2013/066729. Die Verbindungen Q-H mit Q = Q10, Q11, Q12 oder Q13 können beispielweise aus entsprechenden (Het)Aryl-Aminoderivaten durch Ringschluss mit einem Halogenacetaldehyd zur jeweiligen Imidazolverbindung erhalten werden, wie zum Beispiel in WO 2003/099816, WO 2010/083145 oder Organic Process Research & Development 2006, 10, 398-402.

Die Umsetzung der Verbindungen Q-H zu Verbindungen der Formel (IIIa) oder Formel (IIIb) im ersten Verfahrensschritt (Schritt a)) erfolgt in Gegenwart einer Zink-metallorganischen Base der Struktur (NR³R⁴)-Zn-R² oder (NR³R⁴)₂-Zn, in welcher (Ausgestaltung B-1)
R² wie vorstehend (Ausgestaltung 1) definiert ist (daher für Halogen oder -O-Pivaloyl steht),
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl, Ethyl, n-Propyl und i-Propyl.

Bevorzugt ist, dass (Ausgestaltung B-2)
R² wie vorstehend als bevorzugt (Ausgestaltung 2) definiert ist (daher für Halogen steht, insbesondere für Chlor, Brom oder Iod),
R³ und R⁴ gemeinsam eine -(CH₂)₅-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl und Ethyl.

Besonders bevorzugt ist, dass (Ausgestaltung B-3)
R² wie vorstehend als besonders bevorzugt (Ausgestaltung 3) oder als ganz besonders bevorzugt (Ausgestaltung 4) definiert ist (daher für Chlor steht) und
R³ und R⁴ gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR³R⁴) Tetramethylpiperidin (TMP) gemäß Formel (IV).

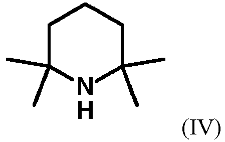

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (V) auf (Ausgestaltung B-4)

(V) (TMP)ₓ ZnCl₂₋ₓ,

worin x für die Zahl 1 oder 2 steht. Hierunter wiederum bevorzugt sind Basen mit x=1 (Ausgestaltung B-5) gemäß Formel (VI):

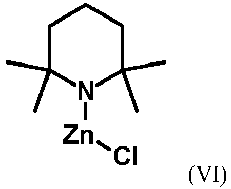

In einer weiteren bevorzugten Ausführungsform des erfindungsgemäßen Verfahrens liegt die Zink-metallorganische Base in Verbindung mit Alkali- oder Erdalkalihalogeniden vor. Dies gilt insbesondere für Basen der Formeln (V) und (VI). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte Zink-metallorganische Basen sind demnach TMP ZnCl·LiCl oder (TMP)₂ Zn·2LiCl (Ausgestaltung B-6). Am meisten bevorzugt ist TMP ZnCl·LiCl (VII; Ausgestaltung B-7)

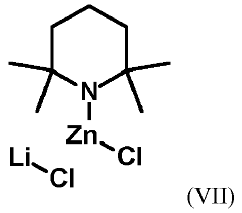

Beispielhaft werden in nachfolgender Tabelle 1 konkrete Kombinationen von Verbindungen der Formeln (I), (II) und (IIIa) bzw. (IIIb) mit erfindungsgemäßen Basen genannt, die in einem erfindungsgemäßen Verfahren zur Anwendung kommen können. Da in einigen Ausgestaltungen das Strukturelement R² sowohl in der erfindungsgemäßen Base als auch in der Verbindung der Formel (IIIa) vorliegt, gilt für R² jeweils die engste Definition.

**Tabelle 1:**

| Nummer | Verbindungen der Formeln (I), (II) und (IIIa) bzw. (IIIb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |
| 22 | Ausgestaltung 4 | Ausgestaltung B-1 |
| 23 | Ausgestaltung 4 | Ausgestaltung B-2 |
| 24 | Ausgestaltung 4 | Ausgestaltung B-3 |
| 25 | Ausgestaltung 4 | Ausgestaltung B-4 |
| 26 | Ausgestaltung 4 | Ausgestaltung B-5 |
| 27 | Ausgestaltung 4 | Ausgestaltung B-6 |
| 28 | Ausgestaltung 4 | Ausgestaltung B-7 |
| 29 | Ausgestaltung 20 | Ausgestaltung B-1 |
| 30 | Ausgestaltung 20 | Ausgestaltung B-2 |
| 31 | Ausgestaltung 20 | Ausgestaltung B-3 |
| 32 | Ausgestaltung 20 | Ausgestaltung B-4 |
| 33 | Ausgestaltung 20 | Ausgestaltung B-5 |
| 34 | Ausgestaltung 20 | Ausgestaltung B-6 |
| 35 | Ausgestaltung 20 | Ausgestaltung B-7 |
| 36 | Ausgestaltung 21 | Ausgestaltung B-1 |
| 37 | Ausgestaltung 21 | Ausgestaltung B-2 |
| 38 | Ausgestaltung 21 | Ausgestaltung B-3 |
| 39 | Ausgestaltung 21 | Ausgestaltung B-4 |
| 40 | Ausgestaltung 21 | Ausgestaltung B-5 |
| 41 | Ausgestaltung 21 | Ausgestaltung B-6 |
| 42 | Ausgestaltung 21 | Ausgestaltung B-7 |
| 43 | Ausgestaltung 22 | Ausgestaltung B-1 |
| 44 | Ausgestaltung 22 | Ausgestaltung B-2 |
| 45 | Ausgestaltung 22 | Ausgestaltung B-3 |
| 46 | Ausgestaltung 22 | Ausgestaltung B-4 |
| 47 | Ausgestaltung 22 | Ausgestaltung B-5 |
| 48 | Ausgestaltung 22 | Ausgestaltung B-6 |
| 49 | Ausgestaltung 22 | Ausgestaltung B-7 |

Vorzugsweise wird die Zink-metallorganische Base in dem erfindungsgemäßen Verfahren in einer Gesamtmenge von 0,5 bis 5,0 Äquivalenten, vorzugsweise von 0,8 bis 2,0 Äquivalenten, weiter bevorzugt von 1,0 bis 1,5 Äquivalenten und besonders bevorzugt von 1,0 bis 1,2 Äquivalenten, bezogen auf die Verbindung Q-H, eingesetzt. Ein Vorteil des erfindungsgemäßen Verfahrens ist es diesbezüglich, dass die metallorganische Base in nahezu stöchiometrischen Mengen eingesetzt werden kann.

In Abhängigkeit davon, ob in der eingesetzten Zink-metallorganischen Base das Strukturelement (NR³R⁴) einfach oder zweifach vorhanden ist, entstehen Zwischenverbindungen der Formel (IIIa) beziehungsweise der Formel (IIIb) in Verfahrensschritt a).

Die Umsetzung der Verbindungen der Formel (IIIa) beziehungsweise (IIIb) zu Verbindungen der Formel (II) im zweiten Verfahrensschritt (Schritt b)) erfolgt in Gegenwart einer Verbindung der Formel (I) 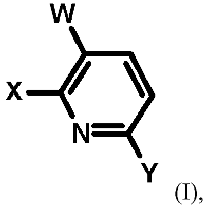 in welcher X, W und Y jeweils die oben genannten Bedeutungen haben.

Die Reaktion findet während der Negishi Kreuzkupplung nahezu ausschließlich an der 2. Position statt, da Brom oder Iod die besten Abgangsgruppen an dem Pyridingerüst sind. Sie liefert dann regioselektiv das entsprechende Pyridinderivat der Formel (II).

Verbindungen der Formel (I) lassen sich beispielsweise durch Substitution einer entsprechenden Vorläuferverbindung, d.h. einem 3-Aminopyridinderivat (vgl. die in Angewandte Chemie International Edition 2015, 54, 7648-7652 oder WO 2017/134066 beschriebenen Verfahren) oder Halogenierung einer entsprechenden Vorläuferverbindung, d.h. einem 2-unsubstituierten Pyridinderivat (vgl. die in WO 2012/112743 beschriebenen Verfahren).

Vorzugsweise wird die Verbindung der Formel (I) in dem erfindungsgemäßen Verfahren in einer Gesamtmenge von 0,5 bis 10,0 Äquivalenten, vorzugsweise von 0,8 bis 5,0 Äquivalenten, weiter bevorzugt von 1,0 bis 2,5 Äquivalenten und besonders bevorzugt von 1,0 bis 1,5 Äquivalenten oder besonders bevorzugt von 1,5 bis 2,0 Äquivalenten oder besonders bevorzugt von 1,0 bis 2,0 Äquivalenten, bezogen auf die Verbindung Q-H, eingesetzt.

Die Umsetzung in Verfahrensschritt b) erfolgt ferner in Gegenwart eines Katalysators. Vorzugsweise ist der Katalysator eine Palladiumverbindung oder eine Nickelverbindung. Besonders bevorzugt ist der Katalysator eine Palladiumverbindung. Ganz besonders bevorzugt handelt es sich um Tetrakis(triphenylphosphine)palladium(0), abgekürzt Pd(PPh₃)₄, der Formel (IX).

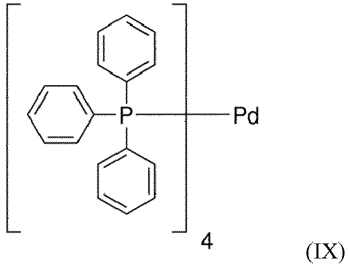

Üblicherweise werden in einem erfindungsgemäßen Verfahren 2,5-25 mol% und vorzugsweise 5-20 mol% Katalysator eingesetzt.
Die erfindungsgemäße Umsetzung der Verbindungen Q-H zu Verbindungen der Formel (IIIa) beziehungsweise (IIIb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrilidon (NEP), N-Butyl-2-pyrrilidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.
Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.
Besonders bevorzugte Lösungsmittel sind THF und N,N-Dimethylformamid (DMF), ganz besonders bevorzugt ist THF.
Das Lösungsmittel kann ferner entgast (sauerstoff-frei) sein.
Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.
Die Umsetzung in Verfahrensschritt a) wird im Allgemeinen bei einer Temperatur zwischen 0 °C und 80 °C und zunehmend bevorzugt zwischen 10 °C und 70 °C, zwischen 15 °C und 60 °C, zwischen 20 °C und 50 °C, zwischen 20 °C und 40 °C und ganz besonders bevorzugt zwischen 20 °C und 35 °C, beispielsweise bei Raumtemperatur bzw. 25 °C, durchgeführt.
Schritt a) erfolgt im Allgemeinen in einem Zeitraum von 5 min bis 12 h, bevorzugt von 15 min bis 10 h und besonders bevorzugt von 30 min bis 2 h.
Die Umsetzung in Verfahrensschritt b) wird im Allgemeinen bei einer Temperatur zwischen 40 °C und 90 °C und zunehmend bevorzugt zwischen 50 °C und 85 °C, zwischen 55 °C und 80 °C, zwischen 60 °C und 80 °C und ganz besonders bevorzugt zwischen 65 °C und 75 °C, beispielsweise bei 65 °C, durchgeführt.
Schritt b) erfolgt in dieser Variante der Erfindung im Allgemeinen in einem Zeitraum von 5 min bis 12 h, bevorzugt von 15 min bis 10 h und besonders bevorzugt von 30 min bis 4 h.
Die Reaktion wird üblicherweise bei Normaldruck durchgeführt, kann aber auch bei erhöhtem bzw. vermindertem Druck durchgeführt werden.
Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.
Ein Beispiel einer besonders bevorzugten Ausführungsform des erfindungsgemäßen Verfahrens kann anhand des folgenden Schemas (II) erläutert werden:

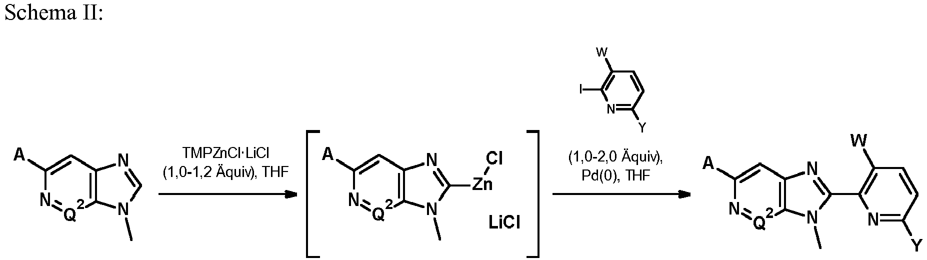

Hierin haben A, Q², W und Y die vorstehend angegebenen Bedeutungen. Die in Klammern angegebene Verbindung stellt die entsprechende Zwischenstufe der Formel IIIa dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. Verbindung der Formel (I). Pd(0) steht für eine Palladiumverbindung als Katalysator, vorzugsweise Pd(PPh₃)₄.

Die vorliegende Erfindung wird anhand der nachfolgenden Beispiele näher erläutert, wobei die Beispiele nicht in einer die Erfindung einschränkenden Weise zu interpretieren sind.

### Analytische Bestimmungen

Die nachstehend beschriebenen Durchführungen der analytischen Bestimmungen beziehen sich auf alle Angaben im gesamten Dokument, sofern die Durchführung der jeweiligen analytischen Bestimmung an der jeweiligen Textstelle nicht gesondert beschrieben ist.

### Massenspektrometrie

Die Bestimmung von [M+H]⁺ oder M⁻ mittels LC-MS unter sauren chromatographischen Bedingungen wurde mit 1 mL Ameisensäure pro Liter Acetonitril und 0,9 mL Ameisensäure pro Liter Millipore-Wasser als Eluenten durchgeführt. Es wurde die Säule Zorbax Eclipse Plus C18 50 mm * 2,1 mm, 1,8 µm verwendet, bei einer Temperatur des Säulenofens von 55 °C.

### Instrumente:

**LC-MS3:** Waters UPLC mit SQD2 Massenspektrometer und SampleManager Probenwechsler. Linearer Gradient 0,0 bis 1,70 Minuten von 10% Acetonitril zu 95% Acetonitril, von 1,70 bis 2,40 Minuten konstant 95% Acetonitril, Fluss 0,85 mL/min.

**LC-MS6 und LC-MS7:** Agilent 1290 LC, Agilent MSD Massenspektrometer, HTS PAL Probenwechsler. Linearer Gradient 0,0 bis 1,80 Minuten von 10% Acetonitril zu 95% Acetonitril, von 1,80 bis 2,50 Minuten konstant 95% Acetonitril, Fluss 1,0 mL/min).

Die Bestimmung von [M+H]⁺ mittels LC-MS unter neutralen chromatographischen Bedingungen wurde mit Acetonitril und Millipore-Wasser mit 79 mg/L Ammoniumcarbonat als Eluenten durchgeführt.

### Instrumente:

**LC-MS4:** Waters IClass Acquity mit QDA Massenspektrometer und FTN Probenwechsler (Säule Waters Acquity 1,7 µm 50 mm * 2,1 mm, Säulenofentemperatur 45 °C). Linearer Gradient 0,0 bis 2,10 Minuten von 10% Acetonitril zu 95% Acetonitril, von 2,10 bis 3,00 Minuten konstant 95% Acetonitril, Fluss 0,7 mL/min.

**LC-MS5:** Agilent 1100 LC System mit MSD Massenspektrometer und HTS PAL Probenwechsler (Säule: Zorbax XDB C18 1,8 µm 50 mm * 4,6 mm, Säulenofentemperatur 55 °C). Linearer Gradient 0,0 bis 4,25 Minuten von 10% Acetonitril zu 95% Acetonitril, von 4,25 bis 5,80 Minuten konstant 95% Acetonitril, Fluss 2,0 mL/min.

Die Retentionzeit-Indizes wurden in allen Fällen aus einer Kalibrierungsmessung einer homologen Serie von geradkettigen Alkan-2-onen mit 3 bis 16 Kohlenstoffen bestimmt, wobei der Index des ersten Alkanons auf 300, der des letzten auf 1600 gesetzt und zwischen den Werten aufeinanderfolgender Alkanone linear interpoliert wurde.

### logP-Werte

Die Bestimmung der logP-Werte erfolgte gemäß EEC Directive 79/831 Annex V.A8 durch HPLC (High Performance Liquid Chromatography) an einer Phasenumkehrsäule (C18) mit Hilfe folgender Methoden:
Der logP[a] Wert wird durch LC-UV Messung im sauren Bereich bestimmt, mit 0,9 mL/L Ameisensäure in Wasser und 1,0 mL/L Ameisensäure in Acetonitril als Eluenten (linearer Gradient von 10% Acetonitrile bis 95% Acetonitril).

Der logP[n] Wert wird durch LC-UV Messung im neutralen Bereich bestimmt, mit 79 mg/L Ammoniumcarbonat in Wasser und Acetonitril als Eluenten (linearer Gradient von 10% Acetonitril bis 95% Acetonitril).

Die Kalibrierung wurde mit einer homologen Reihe geradkettiger Alkan-2-one (mit 3 bis 16 Kohlenstoffatomen) mit bekannten logP Werten durchgeführt. Die Werte zwischen aufeinanderfolgender Alkanone werden durch lineare Regression bestimmt.

Die Messungen der ¹H-NMR Spektren wurden mit einem Bruker **Avance III 400 MHz** Spektrometer, ausgestattet mit einem 1,7 mm TCI Probenkopf, mit Tetramethylsilan als Standard (0,00 ppm) von Lösungen in den Lösungsmitteln CD₃CN, CDCl₃ oder d₆-DMSO durchgeführt. Alternativ wurde ein **Bruker Avance III 600** MHz Spektrometer ausgestattet mit einem 5 mm CPNMP Probenkopf oder ein Bruker **Avance NEO 600** MHz Spektrometer ausgestattet mit einem 5 mm TCI Probenkopf für die Messungen verwendet. In der Regel wurden die Messungen bei einer Probenkopftemperatur von 298 K durchgeführt. Sofern andere Messtemperaturen verwendet wurden, wird dies gesondert vermerkt.

Die NMR-Daten werden in klassischer Form (δ-Werte, Multiplettaufspaltung, Anzahl der H-Atome) aangegeben.

Das Lösungsmittel, in welchem das NMR-Spektrum aufgenommen wurde ist jeweils angegeben.

### Beispiel 1

### Synthese von 3-Methyl-6-(trifluormethyl)-2-{3-[(trifluormethyl)sulfanyl]pyridin-2-yl}-3H-imidazo [4,5-c] pyridin:

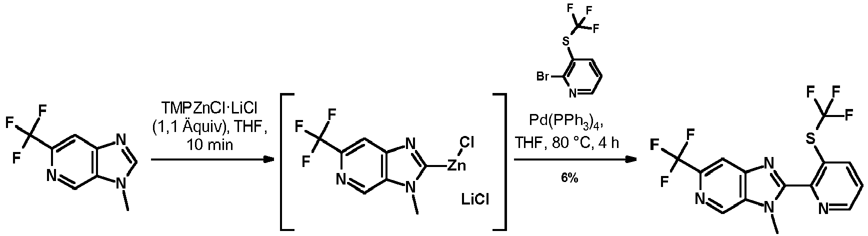

In einem trockenen, mit Argon befüllten Schlenk-Kolben, ausgestattet mit einem Magnetrührfisch und einem Septum, wurde 3-Methyl-6-(trifluormethyl)-3H-imidazo[4,5-c]pyridin (30,0 mg, 0,149 mmol) in wasserfreiem THF (2,0 mL) vorgelegt. Zinkchlorid-2,2,6,6-tetramethylpiperidin-l-id Lithiumchlorid Komplex (TMPZnCl·LiCl) (1,31 M in THF, 0,125 mL, 0,164 mmol) wurde tropfenweise zugegeben, und die Mischung wurde 10 min bei 25 °C gerührt. Eine Lösung von 2-Brom-3-[(trifluormethyl)sulfanyl]pyridin (38,5 mg, 0,149 mmol) in wasserfreiem THF (2,0 mL) sowie Tetrakis(triphenylphosphin)palladium(0) (3,6 mg, 0,015 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[5-(Ethylsulfanyl)-1-methyl-1H-imidazol-4-yl]-7-methyl-3-(trifluormethyl)-7H-imidazo[4,5-c]pyridazin (3,2 mg, 6%) als weißen Feststoff ergab.
HPLC-MS: logP[a] = 3,00; logP[n] = 2,97; MH⁺: 379;
¹H-NMR (d₆-DMSO): δ 9,30 (s, 1H), 8,95 (m, 1H), 8,45 (d, 1H), 8,33 (s, 1H), 7,84 (dd, 1H), 4,09 (s, 3H).

## Patentansprüche

1. Verfahren zur Herstellung von Verbindungen der Formel (II) 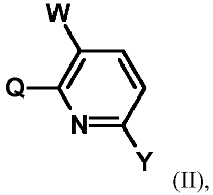 in welcher
Q für ein Strukturelement 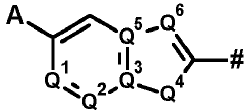
steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N, CR⁶ oder NR⁷ steht,
Q⁵ für N oder C steht,
Q⁶ für N oder CH steht
wobei maximal fünf der Variablen Q¹, Q², Q³, Q⁴, Q⁵ und Q⁶ gleichzeitig für Stickstoff stehen und Q³ und Q⁵ nicht gleichzeitig für N stehen und
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht, oder
A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
W für S(O)ₙR⁸ steht, wobei
R⁸ für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
n für 0, 1 oder 2 steht und
Y für Wasserstoff, Halogen, CO₂R¹ oder NO₂ steht, wobei R¹ für (C₁-C₆)-Alkyl oder (C₁-C₆)-Haloalkyl steht,
**dadurch gekennzeichnet, dass** in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 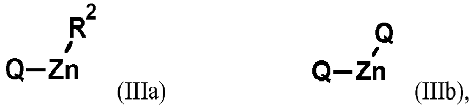 in welcher Q und R² jeweils die oben genannten Bedeutungen haben,
und diese Verbindung der Formel (IIIa) oder (IIIb) in einem zweiten Verfahrensschritt b) mit einer Verbindung der Formel (I) 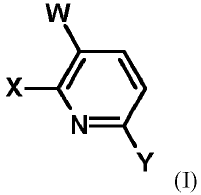 in welcher X für Halogen steht und W und Y jeweils die oben genannten Bedeutungen haben, in Gegenwart eines Katalysators zur Verbindung der Formel (II) umgesetzt wird.

2. Verfahren gemäß Anspruch 1, **dadurch gekennzeichnet, dass**
Q für ein Strukturelement aus der Reihe Q1 bis Q15 steht, wobei 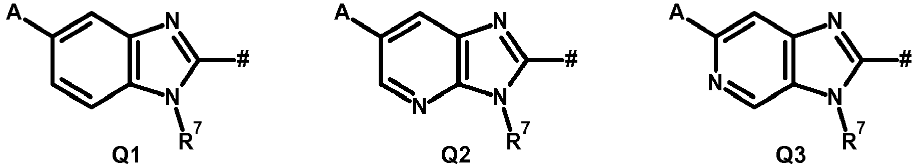 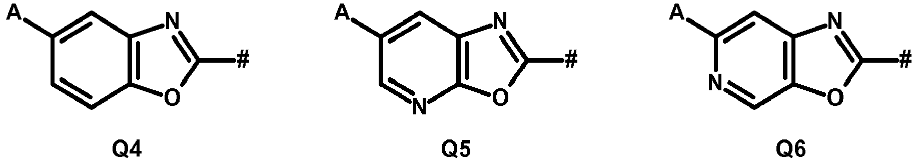 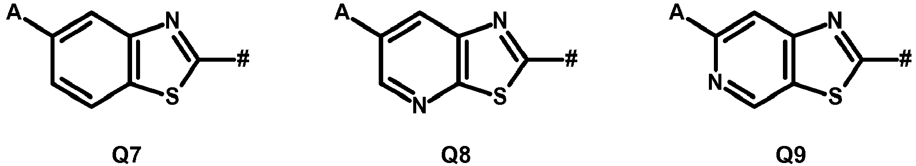 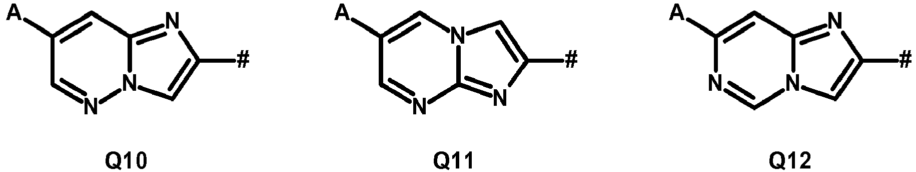 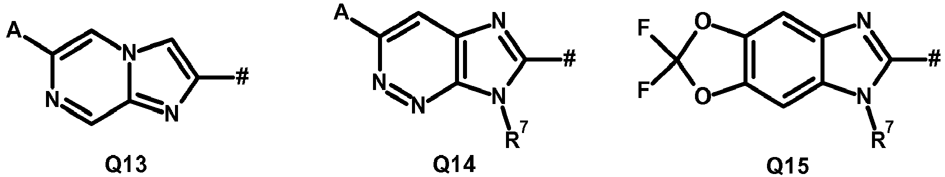
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
W für S(O)ₙR⁸ steht, wobei
R⁸ für (C₁-C₆)Alkyl, (C₁-C₆)Cyanoalkyl, (C₁-C₆)Alkoxy-(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
n für 0, 1 oder 2 steht,
R² für Halogen, insbesondere Chlor, Brom oder Iod steht,
X für Halogen, insbesondere Brom oder Iod steht, und
Y für Wasserstoff, Fluor, Chlor, Brom, CO₂R¹ oder NO₂ steht, wobei R¹ für (C₁-C₄)-Alkyl steht.

3. Verfahren gemäß einem der Ansprüche 1 oder 2, **dadurch gekennzeichnet, dass**
Q für ein Strukturelement aus der Reihe Q2, Q3, Q10, Q12, Q14 oder Q15 steht,
R⁷ für (C₁-C₄)Alkyl oder (C₁-C₄)Alkoxy-(C₁-C₄)alkyl steht,
A für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
W für S(O)ₙR⁸ steht, wobei
R⁸ für (C₁-C₃)Alkyl oder (C₁-C₃)Halogenalkyl steht,
n für 0, 1 oder 2 steht und
R² für Chlor steht,
X für Brom oder Iod, insbesondere Iod steht und
Y für Wasserstoff, Chlor, Brom, CO₂R¹ oder NO₂ steht, wobei R¹ für (C₁-C₄)-Alkyl steht.

4. Verfahren gemäß einem der Ansprüche 1 bis 3, **dadurch gekennzeichnet, dass**
Q für das Strukturelement Q3 oder Q14 steht,
R⁷ für Methyl oder Ethyl, insbesondere Methyl steht,
A für Trifluormethyl steht,
W für S(O)ₙR⁸ steht, wobei
R⁸ für Ethyl oder Trifluormethyl steht und
n für 0 steht,
R² für Chlor steht,
X für Brom oder Iod steht und
Y für Wasserstoff, Chlor, Brom, CO₂R¹ oder NO₂ steht, wobei R¹ für Methyl steht.

5. Verfahren gemäß einem der Ansprüche 1 bis 4, **dadurch gekennzeichnet, dass** R³ und R⁴ gemeinsam eine -(CH₂)₅-Gruppe bilden, die durch 4 Methylgruppen substituiert ist.

6. Verfahren gemäß einem der Ansprüche 1 bis 5, **dadurch gekennzeichnet, dass** es sich bei der Zink-metallorganischen Base um eine Verbindung der Formel (V) handelt
(V) (TMP)ₓ ZnCl₂₋ₓ,
worin x für die Zahl 1 oder 2 steht.

7. Verfahren gemäß einem der Ansprüche 1 bis 6, **dadurch gekennzeichnet, dass** die Zink-metallorganische Base in Verbindung mit einem Alkali- oder Erdalkalihalogenid, vorzugsweise Lithiumchlorid oder Magnesiumchlorid, vorliegt.

8. Verfahren gemäß einem der Ansprüche 1 bis 7, **dadurch gekennzeichnet, dass** die Zink-metallorganische Base in einer Gesamtmenge von 0,5 bis 5,0 Äquivalenten, bezogen auf die Verbindung Q-H, eingesetzt wird.

9. Verfahren gemäß einem der Ansprüche 1 bis 8, **dadurch gekennzeichnet, dass** die Verbindung der Formel (I) in einer Gesamtmenge von 0,5 bis 10,0 Äquivalenten, bezogen auf die Verbindung Q-H, eingesetzt wird.

10. Verfahren gemäß einem der Ansprüche 1 bis 9, **dadurch gekennzeichnet, dass** es sich bei dem Katalysator um eine Palladiumverbindung handelt.

11. Verfahren gemäß einem der Ansprüche 1 bis 10, **dadurch gekennzeichnet, dass** es sich bei dem Katalysator um Tetrakis(triphenylphosphine)palladium(0) handelt.

12. Verfahren gemäß einem der Ansprüche 1 bis 11, **dadurch gekennzeichnet, dass** es in Gegenwart eines Lösungsmittels durchgeführt wird, welches ausgewählt ist aus der Gruppe bestehend aus Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoff, aromatischer Kohlenwasserstoff, Chlorkohlenwasserstoff, Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, 1,2-Dichlorbenzol, Chlortoluol, Trichlorbenzol; 4-Methoxybenzol, fluoriertes Aliphat, fluorierter Aromat, Trichlortrifluorethan, Benzotrifluorid und 4-Chlorbenzotrifluorid, oder einer Mischung aus mindestens zwei dieser Lösungsmittel untereinander.

13. Verfahren gemäß Anspruch 12, **dadurch gekennzeichnet, dass** das Lösungsmittel THF oder N,N-Dimethylformamid (DMF) ist.

14. Verfahren gemäß einem der Ansprüche 1 bis 13, **dadurch gekennzeichnet, dass** Verfahrensschritt a) bei einer Temperatur zwischen 0 °C und 80 °C durchgeführt wird.

15. Verfahren gemäß einem der Ansprüche 1 bis 13, **dadurch gekennzeichnet, dass** Verfahrensschritt b) bei einer Temperatur zwischen 40 °C und 90 °C durchgeführt wird.
